# EUROPEAN PATENT APPLICATION

(11) **EP 1 541 583 A1**
(43) Date of publication of application: **15.06.2005**
(21) Application number: 03739970.6
(22) Date of filing: 26.06.2003
(51) Int. Cl.: C07K 7/06, A61K 38/08, A61P 19/02, A61P 37/06

(54) **NON-T CELL BINDING PEPTIDES AND THEIR USES**

(30) Priority: 27.06.2002 CN 02123412
(71) Applicant: People's Hospital, Peking University, 100044 Beijing (CN)
(72) Inventor: LI, Zhanguo, 100044 Beijing (CN)
(74) Representative: Bublak, Wolfgang
(86) International application number: PCT/CN2003/000496
(87) International publication number: WO 2004/003007

(57) **Abstract**

The present invention provides a non-T cell binding peptide and its analogs for use in the treatment of rheumatoid arthritis. The polypeptide therapeutic agent specifically inhibits the abnormal immune responses of rheumatoid arthritis, focusing on the initiating step of the disease's development, fundamentally controlling the progression of this disease.

## Description

### Field of the Invention

The present invention relates to the preparation of non-T cell binding peptides and their uses as drugs for the treatment of rheumatoid arthritis and other autoimmune diseases.

### Background

Rheumatoid arthritis (RA) is a potentially disabling chronic autoimmune disease characterized mainly by joint destruction and deformity. With a morbidity rate of 0.34% in China, there are nearly five million patients throughout the country with the percentage of disability reaching nearly 93%. The onset process of this disease is an "initiation - linked immune response" driven by antigens. Immune damage mediated by infection factor(s) and autoimmune response(s) is the basis for the onset and progression of rheumatoid arthritis. Antigen polypeptides are expressed through antigen-presenting cells *in vivo,* where they activate T lymphocytes, resulting in the release of cytokines and an increase in the production of inflammatory factors, such as immune globulins, chemokines and free radicals, which consequently induce the typical pathologic changes seen in rheumatoid arthritis, including vasculitis, synovium hyperplasia, and cartilage and bone damage.

Currently, no drug exists which can completely control rheumatoid arthritis. Generally, non-steroidal anti-inflammatory drugs such as Brufen and declofenic acid are used clinically for the temporary relief of symptoms. Disease modifying anti-rheumatoid drugs, such as methotrexate and leflunomide, are used to extensively inhibit immune responses by suppressing DNA synthesis thereby inhibiting joint inflammation. However, in their treatment of rheumatoid arthritis, such drugs do not have an effect on the initial step in the onset of the disease, and, as it is very difficult for them to control the disease specifically, the damage the disease causes continues to progress both systemically as well as in the joints, the end result being disability. Moreover, because of the extensive immune suppression actions of these drugs, they give rise to many side effects, including myelosuppression and abnormal hepatic function, and therefore these drugs cannot be administered to many patients.

At present, there is an urgent need for drugs which work on the underlying pathology of rheumatoid arthritis and treat this disease by suppressing the initial step of immune responses. Following many years of research, the inventors discovered non-T cell binding peptides which can be used for treating rheumatoid arthritis. It is proven in our research that the peptides of the present invention can inhibit the recognition of HLA-DRβ1 antigen molecules and T cells to the antigens, and thereby inhibit the consequent autoimmune responses. As such, they can prevent and control the onset and development of the disease at a key step in the onset of rheumatoid arthritis.

### Summary of the Invention

One object of the present invention is to provide a non-T cell binding peptide, which can be used to treat rheumatoid arthritis effectively.

Another object of the present invention is to provide the use of said non-T cell binding peptides in the treatment of rheumatoid arthritis.

Finally, the present invention provides the pharmaceutical compositions comprising said non-T cell binding peptides.

The term "non-T cell binding peptide" used herein refers to polypeptides which are produced by altering one or more amino acids in SEQ ID NOs: 1-7 and which can still bind to HLA-DRβ1 after alteration. The methods for altering amino acid sequences are well known in the art.

Generally, the present invention comprises using Alanine (A) or Glycine (G) to replace one or more amino acids that function to bind with T cell receptors and act to stimulate their proliferation on a wild type CII peptide, while preserving the amino acids which function to bind to HLA-DRβ1, thus producing a group of novel CII polypeptide molecules which can only bind to HLA-DRβ1 molecules but which cannot be recognized by T cell receptors, i.e., the seven non-T cell binding peptides listed in the attachment (267A, 268A, 269A, 270A, Mut 269-270, Mut 268-270, Mut 267-270). Their specific sequences and IDs are shown in the Sequence Listing section.

The mutual recognition and binding of the three molecules HLA-DRβ1, the antigen peptide and the T cell receptor is the key step in the abnormal immune responses seen in rheumatoid arthritis. Therefore, the present invention focuses on blocking the binding of T cells to antigens, which is the key underlying pathology of rheumatoid arthritis, and furthermore suppresses the *in vivo* abnormal immune responses in patients with rheumatoid arthritis by utilizing the recognition of T cells to antigens and the competitive inhibition of non-T cell binding peptides to pathogenic antigens, thereby controlling the progress of the onset and development of the disease so as to reach the goal of a fundamental treatment of rheumatoid arthritis.

It has already been proven that amino acids 70-74 in HLA-DRβ1 contain a consensus sequence of QK/RRAA (i.e. Gln-Lys/Arg-Arg-Ala-Ala, glutamine - lysine/arginine-arginine-alanine-alanine)⁽¹⁾. This consensus sequence is related to the formation of the HLA-DRβ1-antigen binding cleft and the amino acids in this sequence function in the binding to the antigens. Much research by the inventors and Wucherfennig *et al* has proven that the positively charged amino acid 71 (Lys or Arg) in this sequence is the key site of antigen binding ⁽²⁻⁸⁾.

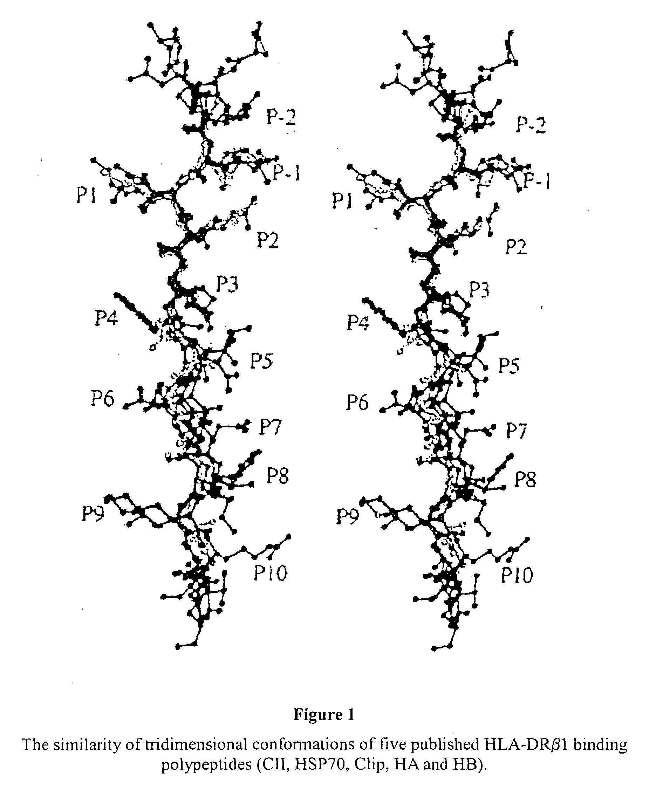

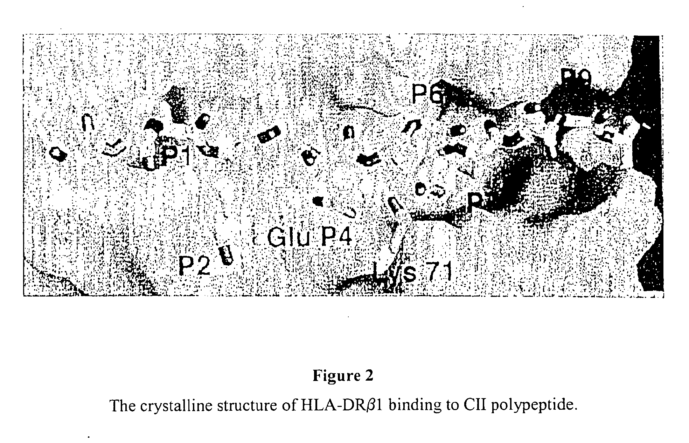

Through research into the crystalline structure of the HLA-DRβ1-antigen dimer using X-ray diffraction technique, it was found that a variety of antigen peptides which bind to rheumatoid arthritis related HLA-DRβ1 (DR4/DR1) molecules are extremely similar in configuration, including denatured type II collagen (CII) and Heat Shock Protein (HSP) ⁽⁹⁻¹²⁾. From the tridimensional structures of these peptides (Figure 1), it can be seen that the side chains of Phe₂₆₃ (P1), Glu₂₆₆ (P4) and Gly₂₇₁ (P9) stretch to the HLA-DRβ1 molecule on the left, and are imbedded into the antigen binding cleft entirely or partially, while side chains of other amino acids stretch to another side or the side opposite HLA-DRβ1 (the side containing the T cell receptor) to stimulate T cell activation. From figure 2, it can be seen that the side chains P1, P4, and P9 of the CII polypeptide are imbedded into the antigen binding "pocket" of HLA-DRβ1. Negatively charged P4 (Glu) is adjacent to positively charged amino acid 71 (Lys₇₁) of HLA-DRβ1, forming a high affinity polar binding. It was therefore posited that Glu₂₆₆ might be the key CII polypeptide amino acid which binds to DRβ1 ^{(13,14)}. In further research, it was proven that CII binds to T cell receptors mainly through Phe₂₆₃, Gly₂₆₅, Glu₂₆₆ and Gly₂₇₁, Glu₂₇₂, and thus activates T cells ⁽¹²⁻¹⁴⁾. From this we can see that the major HLA-DRβ1 binding amino acids in CII polypeptide are Phe₂₆₃, Gly₂₆₅, Glu₂₆₆ and Gly₂₇₁, Glu₂₇₂, whilst the major T cell receptor (TCR) binding amino acids are Gln₂₆₇, Gly₂₆₈, Pro₂₆₉ and Lys₂₇₀ (Table 1).

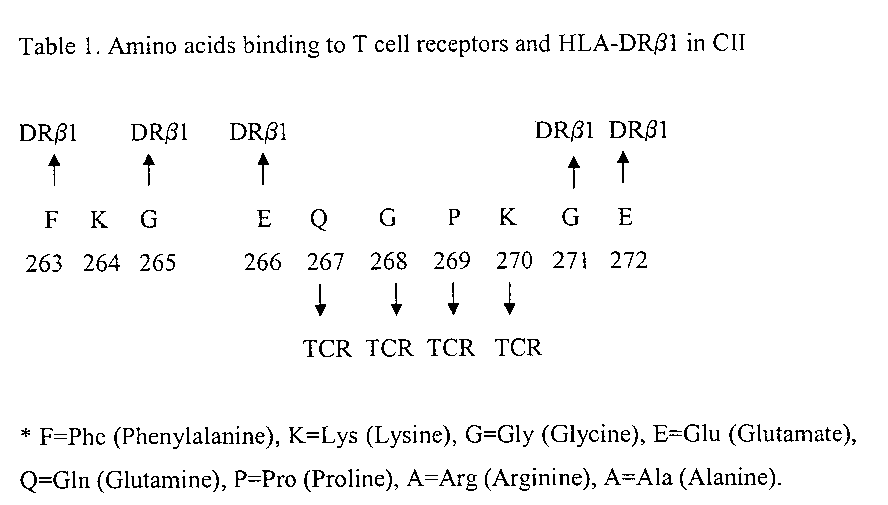

Based on the aforementioned research, the inventors produced a non-T cell binding peptide by substituting the amino acids which bind to the T cell receptors and which act to stimulate T cell proliferation in the CII peptide with Alanine (A) and Glycine (G), and keeping the amino acids which bind to HLA-DRβ. This manufactured non-T cell binding peptide can only bind to DRβ1 molecules and cannot be recognized by T cell receptors. The non-T cell binding peptides specifically bind to HLA-DRβ1, and competitively inhibit the binding of autoantigens to HLA-DRβ1, thus inhibiting HLA-DRβ1 mediated autoimmune responses and their subsequently induced pathologic processes, such as release of inflammatory factors.

For the above reasons, in the embodiments of the present invention, non-T cell binding peptides and their analogs are provided. The following peptides are preferred: F K G E A G P K G E (SEQ ID NO: 1), F K G E Q A P K G E (SEQ ID NO: 2), F K G E Q G A K G E (SEQ ID NO: 3), F K G E Q G P A G E (SEQ ID NO: 4), F K G E Q G A A G E (SEQ ID NO: 5), F K G E Q A G A G E (SEQ ID NO: 6) and F K G E G A G A G E (SEQ ID NO: 7). These polypeptides can bind to the specific sequence QK/RRAA (i.e. Gln - Lys /Arg - Arg -Ala - Ala) in HLA-DRβ1 which is related to the onset of rheumatoid arthritis, and can thereby inhibit T cell activation, and consequently achieve the goal of treating rheumatoid arthritis and other autoimmune diseases mediated by T cells.

In the embodiments of the present invention, compositions comprising said non-T cell binding peptides or their analogs and pharmaceutically accepted carriers or adjuvants are also provided.

The pharmaceutically accepted carriers or adjuvants include lactose, sucrose, sorbic alcohol, mannitol, potato starch, maize starch or amylopectin, cellulose derivatives, glutin, magnesium stearate, calcium stearate and so on. Compositions can be made in the form of tablet, pill, capsule, syrup, powder, granula, liquor and so on. For example, for oral administration of said pharmaceutical composition, the active compound can be mixed with said pharmaceutically accepted carriers or adjuvants, and then compacted into tablets which can be further coated if necessary.

Liquor may also include other excipients well known in the art. The pharmaceutical compositions can also include certain pharmaceutically accepted carriers, such as water, suspension agents, emulsifiers and other ingredients.

The content of the non-T cell binding polypeptides or their analogs in the compositions of the present invention are usually 50-200 µg/tablets and optimally, 100 µg/tablets. A person skilled in the art can determine the specific amount of non-T cell binding polypeptides of the present invention to be administered based on the state of illness, and the body weight and age of a patient.

The non-T cell binding peptides or their analogs of the present invention can be administered to arthritis patients by a variety of methods (oral administration, injection and so on).

### Brief Description of the Drawings

Figure 1: The similarity of the three dimensional conformations of the five published HLA-DRβ1 binding polypeptides (CII, HSP70, Clip, HA and HB). The five overlapping polypeptides are shown in different colors. The conformations of these peptides are shown from different angles in the left and right figures. From the figure, it can be seen that the conformations of these polypeptides are extremely similar. The left side chains of amino acids at position 1 (P1), position 4 (P4) and position 9 (P9) bind to HLA-DRβ1 and are imbedded into the antigen binding clefts either entirely or partially. The side chains of other amino acids stretch to another side or the orientation of TCR, which is opposite to HLA-DRβ1, in order to stimulate T cell activation. (Immunity 7:473-81,1997)
Figure 2: The crystalline structure of HLA-DRβ1 binding to a CII polypeptide. The side chains at P1, P4 and P9 of CII polypeptide are imbedded into the antigen binding "pocket"(s) of HLA-DRβ1. The high affinity polar binding point is formed by the conjunction of (positively charged) Lys₇₁ in HLA-DRβ1 and P4 (Glu, negatively charged) (Immunity 7:473-81,1997).

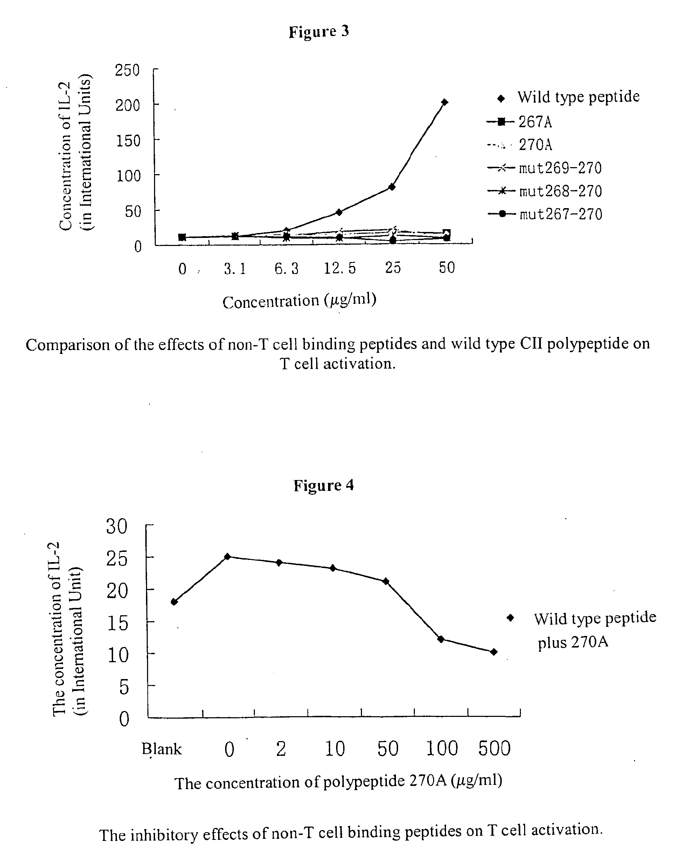

Figure 3: Comparison of the effects of non-T cell binding peptides and wild type CII polypeptide on T cell activation. Compared with the wild type CII peptide, none of the polypeptides Mut 267-270, Mut 268-270, Mut 269-270, 269A or 270A have a significant effect on T cell activation.
Figure 4: The inhibitory effects of non-T cell binding peptides on T cell activation. While co-incubating non-T cell binding peptides and wild type peptides with T cell activation stimulation systems, the T cell activation effects of wild type DRβ1 binding peptides can be inhibited significantly by non-T cell binding peptides. The higher the concentration of non-T cell binding peptides is, the greater the inhibitory effect is.

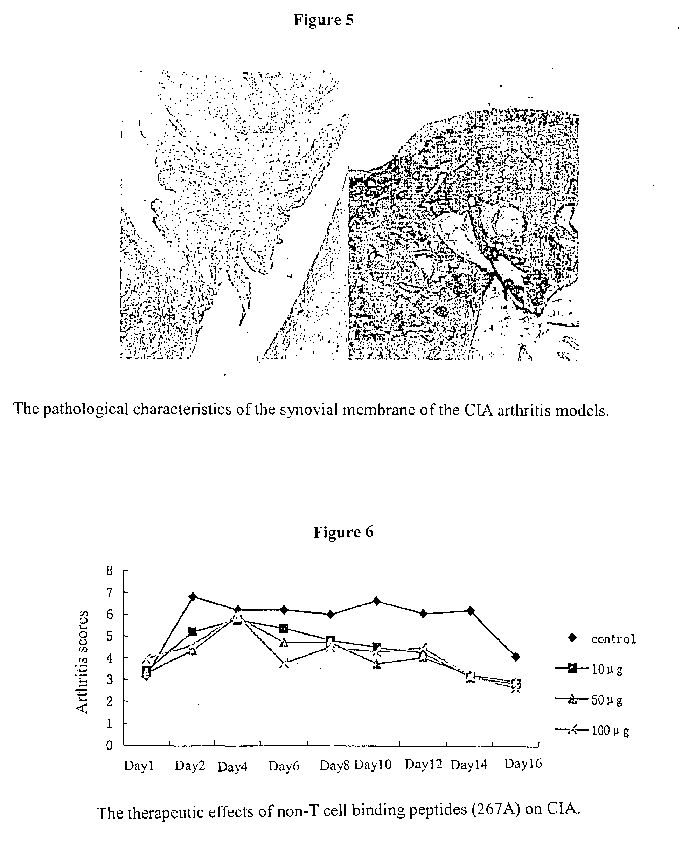

Figure 5: The pathological characteristics of the synovium of CIA arthritis models. Synovial membrane incrassation and lymphocye infiltration were found using HE staining. Pannus formation was also found in some joints. There was a positive correlation between the pathological changes and the ratio of joint swelling.
Figure 6. The therapeutic effects of non-T cell binding peptide (267A) on Collagen-Induced Arthritis (CIA). On Day 8, 10, 12 and 14, the swelling ratio(s) of the right feet and arthritis score(s) of the three treatment groups were all significantly lower than those of the control group (p<0.05 or 0.01). On Day 8 and Day 16 after the treatment, the remission ratio(s) of CIA in the control group were significantly lower than that seen in the three treatment groups (p<0.05).

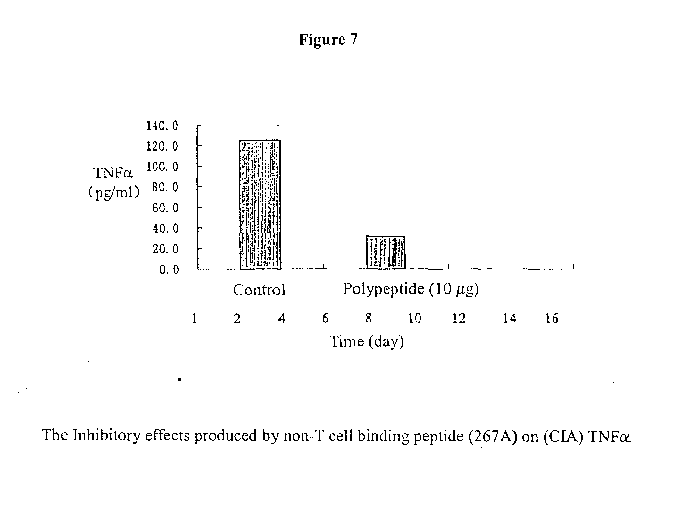

Figure 7. The Inhibitory effects of non-T cell binding peptide (267A) on collagen-induced arthritis (CIA) TNFα. The concentration of TNFα in peripheral blood in 100 µg/ml treated group is significantly lower than that of the control group (p<0.05). No significant variation was found when comparing the other two treatment groups with the control group.

The following examples will be used to further illustrate the present invention, rather than limiting the scope of the present invention.

### Detailed Description of Specific Embodiments

### Example 1 Polypeptide design and synthesis

It has been proven in the research mentioned above ^{(4,5,9-11)} that amino acids in P267-270 in the CII polypeptide mainly bind to T cell receptors and activate T cells. In experiments on the present invention, firstly, a group of seven non-T cell binding peptides containing single or multiple amino acid substitutions (CII 267-270) (table 2) were synthesized using the solid-phase technique reported by Flechsler *et al*.⁽¹⁴⁾. In order to increase the absorption ratio and the bioavailability of these polypeptides and enhance the therapeutic effect, the amino-terminal of each peptide was linked to a myristic acid gene during the process of synthesis to facilitate transportation of the polypeptides into cells. The ten-amino-acid-peptides used in this research all contained four or more hydrophilic residues, including Lysine (K) and Glutamate (E), which are easily dissolved and absorbed. These peptides do not contain methionine (M) or tryptophan (W), which are easily degraded, nor Aspartate (N) from which the amino group or the carboxyl group is prone to be removed. Therefore, the peptides are stable, and it is easy for them to enter into the cells and play a role in disturbing T cell recognition.

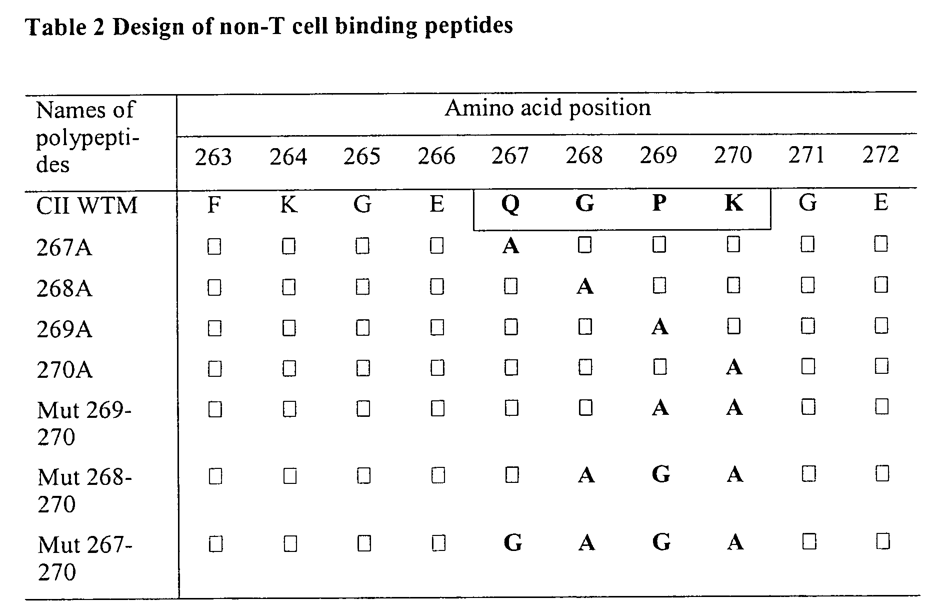

Research indicates that following the substitution of T cell binding amino acids (267-270) with Alanine (A) and/or Glycine (G) respectively, the aforementioned seven non-T cell binding peptides are able to suppress T cell activation. Of such substitutions, cell line tests and rheumatoid arthritis animal models demonstrate that the polypeptides 267A and Mut 267-270 have significant effects (to be described later).

### Example 2 The inhibitory effects of the non-T cell binding peptides of the present invention on T cell activation

Antigen polypeptides are expressed through antigen-presenting cells and activate T cells, resulting in the production of inflammation factors *in vivo,* and further resulting in the typical pathologic changes seen in rheumatoid arthritis. In the experiment, the inhibitory effects of non-T cell binding peptides on T cell activation were detected by measuring T cell proliferation and Interleukin (IL)-2 levels. The following two sets of internationally recognized antigen-presentation and T cell activation systems were used in the research.

| | |
|---|---|
| Antigen-presenting cells | L57.23 cell: recognizing HLA-DR1 antigen (transgened with DR1) |
| | Priess cell: recognizing HLA-DR1/4 (transfected with EBV) |
| T-cells | 3.19 cell: HLA-DR1-specific T cell clone |
| | 3838 cell: HLA-DR4-specific T cell clone |

Within the above, one of the sets of antigen presentation and T cell activation systems was comprised of L57.23 cells and 3.19 cells, while the other was comprised of Priess cells and 3838 cells. By comparing the difference in the effects of said seven non-T cell binding peptides on antigen presentation and T cell activation, non-T cell binding peptides which have significant inhibitory effect(s) on T cell activation were screened out.

Firstly, the antigen-presenting cells, different non-T cell binding peptides (10 µg/ml) and corresponding T cells were added into the reaction system and co-incubated at 37°C for 48 hours. A supernatant was obtained and added to good growth status IL-2 dependent cells (CTLL). The CTLL cell proliferation was measured using the titrazolium salt method (MTT) and in this way the inhibitory effects of the non-T cell binding peptides on T cell activation were determined.

The results of this experiment showed that the wild type DRβ1 binding peptide was able to stimulate T cell proliferation at a concentration of 400 µg/ml, 200 µg/ml, 80 µg/ml, 40 µg/ml and 20 µg/ml, and high concentrations of IL-2 could be detected in the supernatants. However, the stimulatory effects of the seven non-T cell binding peptides on T cell activation were significantly weaker and the concentrations of IL-2 in the supernatants were significantly lower than that of the wild type peptide (p<0.01) (Figure 3). When the non-T cell binding peptides were co-incubated with wild type peptides and a T cell activation system, the non-T cell binding peptides significantly inhibited the T cell activation effects of the wild type DRβ1 binding peptides. Moreover, the inhibitory effect increased as the concentration of non-T cell binding peptides increased (Figure 4). Further tests proved that when using non-T cell binding amino acid 267 substituted polypeptides to treat collagen-induced arthritis rats, the joint swelling and inflammatory reactions were significantly alleviated. The above results show that the wild type CII263-272 polypeptide contains an antigen epitope of Collagen type II, and non-T cell binding peptides produced by substituting T cell binding amino acids of CII263-272 can block the recognition of T cell receptors to that polypeptide and thereof inhibit T cell activation. All these results suggest that non-T cell binding peptides might lead to a new method of interference in the autoimmune responses mediated by HLA-DR1/DR4.

### Example 3 The inhibitory effects of non-T cell binding peptides of the present invention on experimentally induced arthritis

During the immune responses that lead to arthritis, antigen-presenting cells induce T cell activation through antigen(s) and promote T cell proliferation, increase the *in vivo* cytokine (e.g.TNFα, IL-1) level in animals and thereby lead to the typical pathological changes seen in rheumatoid arthritis. Because of this, the inhibitory effects of non-T cell binding peptides on arthritis can be determined by measuring the level of *in vivo* cytokines in experimental animals. At the same time, from a histological perspective, the therapeutic effects can also be evaluated via a pathological section (slice).

In the animal experiment protocol of the present invention, collagen-induced arthritis (CIA) in Wistar rats was induced by bovine CII. Following the use of the non-T cell binding peptides of the present invention to treat the arthritic rats, it was found that polypeptide 267A could significantly suppress the arthritis induced in the rats, alleviating the swelling of the joints and shortening the course of the disease (see the results).

### (1) The establishment of experimentally induced arthritis animal models:

The experimentally induced arthritis model used in the present invention is the internationally recognized collagen-induced arthritis (CIA) model. The experimental animals used were closed colony American Wistar rats, which came from a strain successfully cultivated by Wistar Institute of the USA in 1907. They are among the earliest such species introduced into our country and have been the most widely used. The rats used in this experiment were all cultivated in the animal center of the People's Hospital, Peking University (SPF degree II). The rats were cultured in polyacylene cages cushioned with serrago. The light/dark cycle was maintained as 12h/12h and water and food were freely available to the rats. All the rats used in the experiment were male, 8-10 weeks of age with a body weight of 180±10g. All the protocols related to animals in the experiment were in accordance with the stipulations in the *Chinese Experimental Animal Regulations*.

The bovine type II collagen (catalogue number: C1188) was purchased from Sigma Co. Ltd.. It was dissolved in acetic acid to make a solution with a final concentration of 8 mg/ml. This solution was then emulsified with complete Freund's adjuvant (Difco, Detroit, USA) of equal volume. 100 µl of the emulsified solution was injected into the right footpad of each Wistar rat. The injection dose was 400 µl/rat. In this way the Wistar rat arthritis model was set up.

### (2) Evaluation of the arthritis:

Swelling of metatarsophalangeal joints and interphalangeal joints was found on all the rats about 15 days after the induction of arthritis with CII in the Wistar rats. A 0-16 scale was used to evaluate the extent of arthritis. Each of the 4 paws was assigned a score using a 0-4 scale, wherein zero = no joint swelling, 1 = swelling of one joint, 2 = swelling of 2 joints, 3 = swelling of 3 joints, and 4 = swelling of 4 joints, i.e. swelling of the whole paw. Each of the four paws was scored respectively, and these scores were summed up to give a total score. Beginning 2 days after the immunization and continuing until the conclusion of the experiment, the rats were inspected daily.

### (3) Treatment of arthritis in the Wistar rats with the non-T cell binding peptide(s):

From Day 4 to Day 7 after arthritis had appeared in the rats, the arthritic rats were randomly divided into 4 groups, that is, 3 treatment groups and one control group. Each group consisted of 10 rats and every 3 days, solution of non-T cell binding peptide 267A or sterile distilled water was subcutaneously injected into the base of the tail of each rat. The non-T cell binding peptide 267A was dissolved in sterile distilled water before injection. The concentrations used in the 3 treatment groups were 100 µg/ml, 500 µg/ml and 1000 µg/ml respectively: 100 µl was given to each rat, with the control group receiving sterile distilled water only. The volume change of the right foot of the rats was measured every day by the water-expellation method. Using the aforementioned method of arthritis evaluation, daily arthritis scores were given based on the number of joints with afflicted with arthritis. The rats were killed 18 days after treatment and their peripheral blood was collected. The serum concentrations of the respective cytokines were detected using TNFα and IL-2 ELISA kits (JingMei Biotech Co. Ltd, ShenZhen, P.R.C). In the meanwhile, the joints of the right foot were detached, fixed, decalcified, sectioned and subjected to HE staining.

### (4) Statistical analysis was carried out using SPSS software package.

### (5) Results: The inhibitory effects of the non-T cell binding peptides of the present invention on experimentally induced arthritis.

In an effort to induce arthritis, 40 male Wistar rats were subjected to type II bovine collagen. As a result, varying severities of metatarsophalangeal and interphalangeal joint swelling appeared in all the rats from Day 14 to Day 17. From Day 4 to Day 7 after the appearance of arthritis, the rats were randomly divided into 4 groups, one of which was the control group, while the other three were treatment groups. There were 10 rats in each group. The polypeptide 267A concentrations used in the 3 experimental groups were 100 µg /ml, 500 µg/ml and 1000 µg/ml respectively. Using the aforementioned method of arthritis evaluation, scores were given based on the numbers of joints afflicted with arthritis.

As shown in the results, prior to the treatment, no significant difference in arthritis scores on the ratio of right foot swelling or the concentration of IL-2 in the peripheral blood was observed between the groups. HE staining of all the swollen ankles and metatarsophalangeal joints showed varying degrees of synovial membrane hyperplasia and lymphocyte infiltration, and pannus formation was found in some joints (Figure 5). The pathological changes correlated positively with joint swelling. The swelling observed in the right feet of the rats in the 3 treatment groups was alleviated within the first 2 weeks of treatment, while no significant change on the swelling of the right feet was found in the control group.

Following treatment of the rats with arthritis using the non-T cell binding peptide 267A of the present invention, the arthritis of all the rats in the 3 treatment groups began to be significantly alleviated on Day 4 of the drug administration (Figure 6, Table 3 as seen below). The remission ratios of the 3 treatment groups were significantly higher than those of the control group (P<0.05). On Day 8, 10, 12 and 14, both the right foot swelling ratios and the arthritis scores of the 3 treatment groups were significantly lower than those of the control group (P<0.01); no significant difference among the 3 treatment groups was observed. The TNFα concentrations in the peripheral blood of the 100 µg/ml treated group were significantly lower than those of the control group (P<0.05, Figure 7). All the results prove that non-T cell binding peptides can reduce auto-immune inflammation in rats with arthritis, inhibit the severity and the disease course of collagen-induced arthritis, and furthermore, indicate that the peptides might have a therapeutic effect on rheumatoid arthritis.

**Table 3.**

| The changes of right hind foot swelling in rats of different groups | | | | | | | | | | |
|---|---|---|---|---|---|---|---|---|---|---|
| Number | | Ankle | Toe1 | Toe2 | Toe3 | Toe4 | Toe5 | Hind foot volume (ml) | number of swollen joints | Average |
| Control group | 1 | + | + | + | + | + | + | 1.5 | 6 | 6.2 |
| | 2 | - | + | ++ | ++ | ++ | - | 1.4 | 7 | |
| | 3 | + | ++ | ++ | + | + | - | 1.5 | 8 | |
| | 5 | + | - | ++ | + | + | + | 1.3 | 6 | |
| | 6 | + | - | + | + | + | ++ | 1.4 | 6 | |
| | 7 | + | - | + | ++ | + | + | 1.3 | 6 | |
| | 8 | + | ++ | - | + | + | + | 1.5 | 6 | |
| | 9 | + | + | + | + | + | + | 1.4 | 7 | |
| | 10 | + | + | + | - | + | - | 1.2 | 4 | |
| 10mg group | 1 | + | - | - | + | - | - | 1.2 | 2 | 3.15* |
| | 2 | - | + | + | - | + | + | 1.5 | 4 | |
| | 3 | + | + | - | - | - | + | 1.4 | 3 | |
| | 4 | - | - | - | - | + | ++ | 1.3 | 3 | |
| | 5 | + | - | + | - | - | + + | 1.3 | 4 | |
| | 6 | + | - | + | - | - | - | 1.3 | 2 | |
| | 7 | - | + | - | - | - | - | 1.1 | 1 | |
| | 8 | + | + | ++ | - | + | + | 1.3 | 6 | |
| | 9 | + | - | - | - | - | - | 1.3 | 1 | |
| | 10 | + | ++ | + | - | - | - | 1.2 | 4 | |
| 50mg group | 1 | + | + | + | - | + | - | 1.4 | 4 | 3.25* |
| | 2 | + | + | - | ++ | + | + | 1.4 | 6 | |
| | 3 | + | + | + | - | + | - | 1.4 | 4 | |
| | 4 | - | + | - | + | ++ | ++ | 1.7 | 6 | |
| | 5 | + | - | - | - | + | - | 1.3 | 2 | |
| | 6 | + | - | - | - | - | - | 1.4 | 1 | |
| | 7 | + | - | - | - | - | - | 1.1 | 1 | |
| | 8 | - | - | + | - | - | - | 1.6 | 1 | |
| | 9 | + | + | - | - | - | + | 1.6 | 3 | |
| | 10 | - | - | - | + | + | + | 1.4 | 3 | |

| | | | | | | | | | | |
|---|---|---|---|---|---|---|---|---|---|---|
| * The number of swollen joints significantly reduced compared with the control group (p<0.01). | | | | | | | | | | |

The applicant also performed the aforementioned experimental research of T cell activation assay and/or CIA models with other non-T cell binding peptides of the present application, including 268A, 269A, 270A, Mut 267-270 and Mut 267-270, and the results achieved were similar to the results of 267A.

### References

1 Gregersen P. K, *et al*.: Arthritis Rheum 30: 1250-1213, 1987.
2 Wucherpfennig KW *et al*.: J Exp Med. 181:1597-1600, 1995.
3 Zhanguo Li *et al*.: Chinese Journal of Immunology, 2002, 18: 76-78.
4.Li ZG *et al*.: J Clin Invest (submitted) 2001.
5 Li ZG *et al*.: Chin Med J 2002.
6 Zhanguo Li *et al*.: Clinical Chinese Journal of Internal Medicine 2001, 40: 19-21.
7 Zhanguo Li *et al*.: National Medical Journal of China 2001, 81: 111-113.
8 Zhanguo Li *et al*.: Clinical Chinese Journal of Rheumatology 2001, 5: 145-147.
9 Stem LJ, *et al*.: Nature 368: 215-221, 1994.
10 Fremont DH *et al*.: Science 272: 1001-1004, 1996.
11 Jardetzky TS, *et al*.: Proc. Natl. Acad. Sci.USA93:734-738, 1996.
12 Dessen A, *et al*.: Immunity 7:473-81,1997.
13 Rosloniec EF, *et al*.: J Exp Med.185: 1113-1122, 1997.
14 Anderson EC, *et al*.: Proc. Natl. Acad. USA 95: 7574-79, 1998.
15 Flechsler I *et al*.: J Pept Scil (3): 141-200, 1995.

## Claims

1. A non-T cell binding peptide and analogues thereof which bind to the target sequences having the consensus sequence QK/RRAA (i.e. Gln -Lys/Arg -Arg -Ala -Ala).

2. A non-T cell binding peptide which is selected from the group consisting of the following sequences:

FKGEAGPKGE (SEQ ID NO: 1)

FKGEQAPKGE (SEQ ID NO: 2)

FKGEQGAKGE (SEQ ID NO: 3)

FKGEQGPAGE (SEQ ID NO: 4)

FKGEQGAAGE (SEQ ID NO: 5)

FKGEQAGAGE (SEQ ID NO: 6)

FKGEGAGAGE (SEQ ID NO: 7).

3. A pharmaceutical composition comprising the non-T cell binding peptide(s) and analogues thereof according to Claim 1, and pharmaceutically accepted carriers and adjuvants.

4. The use of the non-T cell binding peptide(s) and analogues thereof according to Claim 1 in the preparation of a medicament for the treatment of rheumatoid arthritis.

5. The use of the non-T cell binding peptide(s) and analogues thereof according to Claim 1 in the treatment of rheumatoid arthritis.

6. Administration of the non-T cell binding peptide(s) and analogues thereof according to Claim 1 to patients with rheumatoid arthritis.

7. A pharmaceutical composition comprising the non-T cell binding peptide(s) according to Claim 2, and pharmaceutically accepted carriers and adjuvants.

8. The use of the non-T cell binding peptide(s) according to Claim 2 in the preparation of a medicament for the treatment of rheumatoid arthritis.

9. The use of the non-T cell binding peptide(s) according to Claim 2 in the treatment of rheumatoid arthritis.

10. Administration of the non-T cell binding peptide(s) according to Claim 2 to patients with rheumatoid arthritis.


Sequence Listing

<110> People's Hospital, Peking University

<120> Non-T Cell Binding Peptides and Their Uses

<130>

<140> PF030044
<141> 2002-06-22

<160> 7

<170> PatentIn version 3.1

<210> 1
<211> 10
<212> PRT
<213> Artificial sequence

<220>
<223> Synthesized

<400> 1
Phe Lys Gly Glu Ala Gly Pro Lys Gly Glu
1               5                   10

<210> 2
<211> 10
<212> PRT

<213> Artificial sequence

<220>
<223> Synthesized

<400> 2
Phe Lys Gly Glu Gln Ala Pro Lys Gly Glu
1               5                   10

<210> 3
<211> 10
<212> PRT
<213> Artificial sequence

<220>
<223> Synthesized

<400> 3
Phe Lys Gly Glu Gln Gly Ala Lys Gly Glu
1               5                   10

<210> 4
<211> 10
<212> PRT
<213> Artificial sequence

<220>
<223> Synthesized

<400> 4
Phe Lys Gly Glu Gln Gly Pro Ala Gly Glu
1               5                   10

<210> 5
<211> 10
<212> PRT
<213> Artificial sequence

<220>
<223> Synthesized

<400> 5
Phe Lys Gly Glu Gln Gly Ala Ala Gly Glu
1               5                   10

<210> 6
<211> 10
<212> PRT
<213> Artificial sequence

<220>
<223> Synthesized

<400> 6
Phe Lys Gly Glu Gln Ala Gly Ala Gly Glu
1               5                   10

<210> 7
<211> 10
<212> PRT
<213> Artificial sequence

<220>
<223> Synthesized

<400> 7

Phe Lys Gly Glu Gly Ala Gly Ala Gly Glu
1               5                   10